# EUROPEAN PATENT SPECIFICATION

(11) **EP 2 359 824 B1**
(45) Date of publication and mention of the grant of the patent: **05.08.2020**
(21) Application number: 10195669.6
(22) Date of filing: 17.12.2010
(51) Int. Cl.: A61K 35/08, A61K 36/752, A61K 36/61, A61K 36/53, A61K 36/54, A61P 31/00

(54) **Multiplication of the efficacy of anti-infectious agents by a composition comprising an extract made from dehydrated and partially sodium-free seawater**
Multiplikation der Effizienz von antiinfektiösen Wirkstoffen durch eine Zusammensetzung mit einem Extrakt aus dehydriertem und teilweise natriumfreiem Meerwasser
Multiplication de l'efficacité d'agents anti-infectieux par une composition comprenant un extrait à base d'eau de mer déshydratée et partiellement désodée

(30) Priority: 23.02.2010 EP 10305180
(43) Date of publication of application: 24.08.2011
(73) Proprietor: Lagny, Pierre, County Kildare (IE)
(72) Inventor: Lagny, Pierre, Co. Kildare (IE); Herault, Jean-Jacques, London E130LJ (GB)
(74) Representative: Novagraaf Technologies

(56) References cited:
- EP-A1- 0 308 564
- EP-A1- 0 310 476
- EP-A2- 2 258 339
- DE-U1-202004 012 980
- HAMMER K A ET AL: "ANTIMICROBIAL ACTIVITY OF ESSENTIAL OILS AND OTHER PLANT EXTRACTS", JOURNAL OF APPLIED MICROBIOLOGY, OXFORD, GB, vol. 86, no. 6, 1 January 1999 (1999-01-01), pages 985-990, XP001108841, ISSN: 1364-5072, DOI: DOI:10.1046/J.1365-2672.1999.00780.X
- DATABASE WPI Week 200966 Thomson Scientific, London, GB; AN 2009-P25747 XP002636332, & JP 2009 221177 A (ANDES DENKI KK) 1 October 2009 (2009-10-01)
- BYRNE C ET AL: "Efficacy of a new quarternary ammonium compound against TB.", IRISH JOURNAL OF MEDICAL SCIENCE 1999 JAN-MAR LNKD- PUBMED:10098344, vol. 168, no. 1, January 1999 (1999-01), pages 45-46, XP002636333, ISSN: 0021-1265

## Description

The present invention relates in general to the destruction and/or the inhibition of living or unicellular organisms such as protozoa, microbes, bacteria, gametes, fungi, yeasts, parasites or others.

Many substances inhibiting or destroying unicellular living organisms are already known, and these include the surfactant agents such as quaternary ammonium salts. In particular, it is known that quaternary ammonium halides such as benzalkonium chloride (or alkyl-benzyl-dimethyl-ammonium chloride), alone or in combination with other active ingredients, are advantageous in these applications (see, for example, British Patents GB 1 554 615, French Patents FR 2 431 859, FR 2 483 177, FR 2 379 508, FR 2 384 497, FR 2 457 641, FR 2 573 624, FR 2 418 221, FR 2 562 888, European Patents EP 0 243 713, EP 0 175 338, EP 0 132 963, EP 0 127 131, EP 0 094 562, EP 0 076 136, EP 0 068 399, EP 0 037 593, and international applications WO 84/00877 and WO 84/02649).

Moreover, the prior art has already provided many processes for manufacturing these quaternary ammonium salts (among quaternary ammonium halides), directly applicable to the chlorides and/or iodides, and/or bromides, and/or chloro-iodites (see above Patents and also French Patents FR 2 472 558, FR 2 033 044 and European Patents EP 0 094 552 and EP 0 012 296).

Ammonium fluoride and processes for preparing and purifying it are also known (see, for example, French Patents FR 2 244 713, 2 253 710 and European Patent EP 0 002 016), as are perfluorinated or polyfluorinated quaternary ammonium salts (for example, French Patents 2 038 421, 2 051 095, 2 153 489 and European Patents EP 0 073 760, EP 0 100 478, EP 0 100 477, and EP 0149 172).

In European Patent EP 0308564, the problem of providing a composition that completely inhibits or destroys unicellular living organisms and which is simultaneously applicable to living organisms, human beings, animals or plants, without thereby causing harmful side effects, is raised. The solution to this problem described in EP 0308564 consists in a composition containing at least a myristalkonium fluoride, which may be used in human beings or animals. However, this composition has the disadvantage of causing an irritation at the site of injection, except when it is used through central administration. This irritation, caused by the necessary amount of active ingredient in the composition, may be improved by the reduction of the amount of this active ingredient when it is combined with an activating agent.

Moreover, European Patent EP 0310476 describes a pharmaceutical composition containing ionic and ionizable fluorine, and ionic or ionizable lithium, in particular lithium fluoride, i.e. a product of chemical origin.

The applicant has discovered that the addition of an extract made from dehydrated and partially sodium-free seawater in a composition comprising an anti-infectious agent, allows in a surprising manner an increase in its inhibiting or destroying potency towards living or unicellular organisms.

On the other hand, such a compound only has by itself a minor potency to inhibit or destroy an infection and it only has a catalytic effect towards the anti-infectious agents used (also called active ingredients in the present application).

The invention is defined by the appended claims.

### Activating agent

The activating agent of the composition according to the invention, as it is an extract made from the dehydration and partial withdrawal of sodium chloride from seawater, essentially contains the 83 known elements which are present in seawater.

The activating agent is preferably present in the composition according to the invention in the amount of 0.01 ppm (or mg/L) to 120 ppm (or mg/L) to obtain the surprising effects described and claimed in the present invention (i.e. a noticeable activation of the inhibiting potency of the active ingredient (or ingredients) present in the composition according to the invention).

### Anti-infectious agent

The anti-infectious agent is chosen among
- the virucides of vegetable origin, which may be chosen among the essential oils of oregano (*Origanum majorana*), thyme (*Thymus vulgaris*), and savory (*Satureja montana*), etc., or among plant extracts such as grapefruit (*Citrus paradisi*) seed extract.

In the present invention, product of vegetable origin means the whole product of the plant or any type of plant extract, whatever the method of extraction or of production.

The composition according to the invention may be used in the fields of surface disinfection, hygiene products or cosmetic products, insofar as they contain an anti-infectious agent, as well as of phyto-sanitary products and food preservative agents.

Hence, the composition according to the invention may contain in addition one or several ingredients (or additional compounds) which are classically used in the concerned fields. The amounts of these various additional ingredients are those usually used in the concerned fields.

Of course, the specialist in the art will make sure to choose the possible compound or compounds to add to the composition according to the invention (in particular according to the envisaged utilization or application), as well as their concentration, so that the avantageous properties intrinsically linked should not be, or should not be substantially altered by the envisaged addition. In particular, the avantageous properties of the anti-infectious agent will not have to be damaged by this (these) additional compound(s).

Thus, the object of the present invention is also to use the composition according to the invention in a cosmetic or body hygiene product.

More generally, the composition according to the invention may be applied to the human or animal body as a whole, or to vegetable organisms, or to inert surfaces to disinfect.

The present invention also relates to the composition according to claim 1 for use in treating infectious diseases of viral, or bacterial or fungal origin, concerning the whole human or animal body.

The addition of the activating agent to said composition results in an increase in the inhibiting or destroying potency of the anti-infectious agent.

This activating agent is preferably present in said composition in the amount of 0.01 mg/L to 120 mg/L.

Such a composition according to the invention allows the activation of the activity of the anti-infectious agent, which may relate to the whole human or animal body.

The importance of the activation may vary according to the microorganisms encountered but not the reality of the effect.

More particularly, such a composition according to the invention allows the activation of a bactericidal activity to combat an infectious agent of the bacterial families. This effect may be applied to all bacterial families: gram-positive and gram-negative cocci, gram-positive and gram-negative bacilli, acid- and alcohol-fast bacilli, spiral bacteria, etc. This utilization for bactericidal purposes allows not only the inhibition of bacterial resistances (for example, in general, beta-lactamase-positive bacteria become beta-lactamase-negative) in increasing the efficacy of the active ingredients, but also allows the reduction of the doses of active ingredients, whatever the active ingredient, and thus it minimizes their possible adverse side effects in human beings, animals, plants and in the environment as a whole.

More particularly, such a composition according to the invention allows the activation of a fungicidal activity, which may be applied to the different forms of fungi: dermatophytes, yeasts, etc.

More particularly, such a composition according to the invention allows the activation of virucidal active ingredients such as tetradecyl-dimethyl-benzyl-ammonium fluoride mentioned above.

A preferred composition according to the invention consists in the addition of an extract made from dehydrated and partially sodium-free seawater (food compound) to an anti-infectious agent of vegetable origin such as grapefruit seed extract, in order to increase the inhibiting potency of this one. Due to its nature, this composition, which contains food compounds, represents an alternative of complete harmlessness to the usually used anti-infectious agents.

The invention is illustrated in greater detail in the following examples. In the examples, except where otherwise stated, all the amounts are expressed as a percentage in volume, or as mg/L, or as µg or as µL of composition.

### EXAMPLES

### Products

### • Microorganisms :

∘ *Staphylococcus aureus* ATCC 29213,
∘ MRSA (meticillin resistant *Staphylococcus aureus*) ATCC 1026,
∘ *Streptococcus pneumoniae* ATCC 49619,
∘ *Escherichia coli* ATCC 25922,
∘ *Pseudomonas aeruginosa* ATCC 27853,
∘ *Acinetobacter baumanii* ATCC 19606,
∘ *Klebsiella pneumoniae* ATCC 13883,
∘ *Serratia marcescens* ATCC 43861,
∘ *Candida albicans,* nosocomial strain,
∘ *Malassezia furfur,* nosocomial strain.

### • Anti-infectious agent tested :

Grapefruit seed extracts (*Citrus paridisi*), called hereafter GSE. Several extracts were used in the examples presented below in order to show the action of the activating agent, whatever the extract.

### • Activating agent:

Extract made from dehydrated and partially sodium-free seawater, called hereafter PAM. It is an extract made from the dehydration and partial withdrawal of sodium chloride from seawater. It essentially contains the 83 known elements which are present in seawater. This activating agent was used in the above-mentioned examples but it may be replaced by any other extract made from a dried fossil sea with a similar composition (example: Himalaya salt).

### Material and method

The aim is to determine the minimal inhibitory concentration (MIC) of an active ingredient (or anti-infectious agent) against eight bacterial strains and two fungal strains, each strain being inserted in an *inoculum,* in the presence of an agent activating the inhibiting potency, according to the present invention.

With regard to the present invention, the minimal inhibitory concentration (MIC) means the minimal concentration (in % vol/vol) of active ingredient necessary to inhibit 100 µL of *inoculum.*

To determine the MIC, the following compositions are prepared for each example :
- a sample of positive control (called letter B in the test tables with bacteria and letter F in the test tables with fungi), which only contains the *inoculum* (100 µL) in the sterile culture medium of the microorganisms (100 µL).
- a sample of negative control (called letter M in the test tables), which only contains the sterile culture medium of the microorganisms (200 µL), and
- one or several inhibiting compositions according to the present invention, Ci (i corresponding to a whole number), containing:
   ∘ 100 µL of a mixture of active ingredient and activating agent, or
   ∘ 100 µL of a mixture of active ingredient and activating agent, with an excipient (dispersing agent),
   to which 100 µL of *inoculum* are added.

The trials were carried out with the eight above-mentioned bacterial strains according to the method of microdilution in liquid medium described by the CLSI (Clinical and Laboratory Standards Institute), which was adapted if necessary. The method may be summarized as follows :
- Distribute 100 µL of sterile culture medium in the pits of positive control of a 96-well, U-bottom plate.
- Add to each appropriate pit of the 96-well plate:
   1. 100 µL of an anti-infectious agent, or
      95 µL of an anti-infectious agent and 5 µL of an activating agent, or
      10 µL of a mixture of an anti-infectious agent and an activating agent in equal parts and 90 µL of an excipient (dispersing agent),
      keeping to a total volume of 100 µL, except in the pits of positive control and negative control.
   2. 100 µL of *inoculum* with an end concentration of 3 - 5 x 10⁵ CFU/mL (colony-forming units per milliliter), except in the pits of negative control.
- Distribute 200 µL of sterile culture medium in the pits of negative control of the 96-well plate.
- The plate is covered and incubated at 37° C for 18 to 24 hours.
- The results are read with a reading-mirror or a spectrophotometer at the optical density of 620 nm.

The sensitivity threshold is not available in the CLSI (Clinical and Laboratory Standards Institute) guide.

The trials were also carried out with the two above-mentioned fungal strains according to the method of microdilution in liquid medium described by the CLSI (Clinical and Laboratory Standards Institute). The sensitivity threshold is not available in the CLSI guide.

These trials were carried out in the Laboratory of medical microbiology, Research and Development Unit of the University Hospital Center, Hôtel Dieu Hospital, Montreal, Canada.

### EXAMPLE 1: activation of the inhibiting activity of grapefruit seed extract (Citrus paradisi) (GSE) on a reference bacterial strain

In addition to the compositions of control described above (B positive control and M negative control), the following compositions were prepared in order to show the action of the activating agent:
- a first comparative inhibiting composition CC₁, containing 100 µL of GSE,
- a second comparative inhibiting composition C₁ according to the invention, containing 95 µL of GSE and 5 µL of PAM, i.e. a total volume of 100 µL,

The final concentration of PAM in the medium, i.e. in the 200 µL of each appropriate pit, is 60 mg/L. Therefore there are 12 µg of PAM in these 200 µL.
- a third comparative inhibiting composition C₂ according to the invention, containing 95 µL of GSE and 5 µL of PAM, i.e. a total volume of 100 µL,

The final concentration of PAM in the medium, i.e. in the 200 µL of each appropriate pit, is 120 mg/L. Therefore there are 24 µg of PAM in these 200 µL.

100 µL of *inoculum* are added to each of the compositions described above.

For each of these compositions, the MIC of the active ingredient (GSE) was determined on a reference bacterial strain. The experimental results of these trials are presented in table 1 below.

**Table 1**

| **Reference bacterial strain** | **Negative control** | **Positive control** | **MIC (% v/v) of the bacterial strain by GSE alone or combined with PAM (60 or 120 mg/L) ¹** | | |
|---|---|---|---|---|---|
| | **M ⁴** | **B ³** | **CC₁** | **C₁** | **C₂** |
| | | | **GSE** | **GSE** | **GSE** |
| (100 µL of *inoculum)* | | | (100 µL = 50 %)² | (95 µL = 47.5 %) ² + **PAM (60 mg/L) ¹** (5 µL = 2.5 %)² | (95 µL = 47.5 %) ² **+ PAM (120 mg/L)¹** (5 µL = 2.5 %) ² |
| *Staphylococcus aureus* ATCC 29213 | - | + | 0.125 | 0.05 | 0.05 |

| | | | | | |
|---|---|---|---|---|---|
| ¹ Final concentrations of PAM in the medium, i.e. 12 µg or 24 µg of PAM in the 200 µL of each appropriate pit. ² Percentage of each product in the 200 µL of each appropriate pit. ³ Positive control (*inoculum*) : 100 % bacterial growth. ⁴ Negative control (sterile culture medium) : 0 % bacterial growth. | | | | | |

The experimental results presented in table 1 show that the addition of the activating agent PAM according to the invention allows an increase in the inhibiting potency of grapefuit seed extract (GSE), the MIC of which is divided by 2.5 (0.05 instead of 0.125) for the microorganism *Staphylococcus aureus* ATCC 29213.

In composition C₁ according to the invention, 12 µg of activating agent PAM are sufficient to increase the efficacy of 95 µL of grapefuit seed extract (GSE). Therefore, in composition C₁, an amount of PAM approximately 7 916 times lower than that of GSE is sufficient to increase the efficacy of this one.

In composition C₂ according to the invention, 24 µg of activating agent PAM are sufficient to increase the efficacy of 95 µL of grapefuit seed extract (GSE). Therefore, in composition C₂, an amount of PAM approximately 3 958 times lower than that of GSE is sufficient to increase the efficacy of this one.

In this example, the utilization of the activating agent PAM at a dose of 120 mg/L in composition C₂ did not yield a MIC of GSE lower than that observed with 60 mg/L in composition C₁: the MICs of C₁ and C₂ are 2.5 times lower than that of composition CC₁ (0.05 instead of 0.125). Therefore, the efficacy of the activating agent PAM according to the invention is not dose-dependent, but it is effective within precise limits.

These experimental results show that the inhibiting compositions according to the invention allow an increase in the inhibiting potency of the active ingredient used.

### EXAMPLE 2: activation of the inhibiting activity of grapefruit seed extract (Citrus paradisi) (GSE) on seven reference bacterial strains

In addition to the compositions of control described above (B positive control and M negative control), the following compositions were prepared in order to show the action of the activating agent:
- a first comparative inhibiting composition CC₁, containing 100 µL of GSE,
- a second comparative inhibiting composition C₁ according to the invention, containing 95 µL of GSE and 5 µL of PAM, i.e. a total volume of 100 µL,

The final concentration of PAM in the medium, i.e. in the 200 µL of each appropriate pit, is 60 mg/L. Therefore there are 12 µg of PAM in these 200 µL.

100 µL of *inoculum* are added to each of the compositions described above.

For each of these compositions, the MIC of the active ingredient (GSE) was determined on seven reference bacterial strains. The experimental results of these trials are presented in table 2 below.

**Table 2**

| **Reference bacterial strains** (100 µL of *inoculum*) | **Negative control** | **Positive control** | **MIC (% v/v) of the bacterial strains by GSE alone or combined with PAM (60 mg/L)¹** | |
|---|---|---|---|---|
| | | | **CC₁** | **C₁** |
| | **M ⁴** | **B ³** | **GSE** (100 µL = 50 %) ² | **GSE** (95 µL = 47.5 %) ² **+ PAM (60 mg/L) ¹** (5 µL = 2.5 %) ² |
| *MRSA* ATCC 1026 | - | + | 0.125 | 0.029 |
| *Streptococcus pneumoniae* ATCC 49619 | - | + | 0.125 | 0.029 |
| *Escherichia coli* ATCC 25922 | - | + | 0.125 | 0.05 |
| *Pseudomonas aeruginosa* ATCC 27853 | - | + | 0.125 | 0.05 |
| *Acinetobacter baumanii* ATCC 19606 | - | + | 0.125 | 0.05 |
| *Klebsiella pneumoniae* ATCC 13883 | - | + | 0.125 | 0.05 |
| *Serratia marcescens* ATCC 43861 | - | + | 0.125 | 0.05 |

| | | | | |
|---|---|---|---|---|
| ¹ Final concentration of PAM in the medium, i.e. 12 µg of PAM in the 200 µL of each appropriate pit. ² Percentage of each product in the 200 µL of each appropriate pit. ³ Positive control (*inoculum*) : 100 % bacterial growth. ⁴ Negative control (sterile culture medium) : 0 % bacterial growth. | | | | |

In this trial on 7 reference bacterial strains (table 2), results similar to those observed in example 1 on *Staphylococcus aureus* ATCC 29213 are observed.

For the microorganisms MRSA (meticillin resistant *Staphylococcus aureus)* ATCC 1026 and *Streptococcus pneumoniae* ATCC 49619, in composition C₁ according to the invention, the addition of the activating agent PAM according to the invention, at a dose of 60 mg/L, allows an increase in the inhibiting potency of grapefruit seed extract (GSE), the MIC of which is divided by 4.3 (0.029 instead of 0.125). This example is of particular interest because the usual antibiotics are ineffective against this microorganism which is resistant, according to its name, and which causes an increasing anxiety in the scientific circles (see 20th European Congress on Clinical Microbiology and Infectious Diseases, Vienne, April 10-13,2010).

For the microorganisms *Escherichia coli* ATCC 25922, *Pseudomonas aeruginosa* ATCC 27853, *Acinetobacter baumanii* ATCC 19606, *Klebsiella pneumoniae* ATCC 13883 and *Serratia marcescens* ATCC 43861, in composition C₁ according to the invention, the addition of the activating agent PAM according to the invention, at a dose of 60 mg/L, allows an increase in the inhibiting potency of grapefruit seed extract (GSE), the MIC of which is divided by 2.5 (0.05 instead of 0.125).

In composition C₁ according to the invention, 12 µg of activating agent PAM are sufficient to increase the efficacy of 95 µL of grapefuit seed extract (GSE). Therefore, in this example, an amount of PAM approximately 7 916 times lower than that of GSE is sufficient to increase the efficacy of this one.

These experimental results show that the inhibiting composition according to the invention allows an increase in the inhibiting potency of the active ingredient used.

### EXAMPLE 3: activation of the inhibiting activity of grapefruit seed extract (Citrus paradisi) (GSE) on a reference bacterial strain

In addition to the compositions of control described above (B positive control and M negative control), the following compositions were prepared in order to show the action of the activating agent:
- a first comparative inhibiting composition CC₂, containing 100 µL of GSE,
- a group of comparative inhibiting composition C₃, C₄, C₅, C₆, according to the invention, containing for a total volume of 100 µL:
   ∘ 5 µL of GSE,
   ∘ 5 µL of PAM, and
   ∘ 90 µL of excipient (dispersing agent or Disper®).

The final concentration of PAM in the medium, i.e. in the 200 µL of each appropriate pit, is 4 mg/L for C₃, 6 mg/L for C₄, 8 mg/L for C₅ and 13 mg/L for C₆. Therefore the amount of PAM in these 200 µL is as follows: 0.8 µg for C₃, 1.2 µg for C₄, 1.6 µg for C₅ and 2.6 µg for C₆.

100 µL of *inoculum* are added to each of the compositions described above.

For each of these compositions, the MIC of the active ingredient (GSE) was determined on a reference bacterial strain. The experimental results of these trials are presented in table 3 below.

**Table 3**

| **Reference bacterial strain** (100 µL of *inoculum*) | **Negative control** | **Positive control** | **MIC (% v/v) of the bacterial strain by GSE alone or combined with PAM (4 or 6 or 8 or 13 mg/L) ¹ and an excipient (dispersing agent or Disper®)** | | | | |
|---|---|---|---|---|---|---|---|
| | | | **CC₂** | **C₃** | **C₄** | **C₅** | **C₆** |
| | **M⁴** | **B³** | **GSE** (100 µL = 50 %) ² | **GSE** (5 µL = 2.5 %) ² **+ PAM (4 mg/L) ¹** (5 µL %) ² = + **Disper** ® (90 µL = 45 %) ² | **GSE** (5 µL = 2.5 %) ² **+ PAM (6 mg/L) ¹** (5 µL %) ² = **+ Disper** ® (90 µL = 45 %)² | **GSE** (5 µL = 2.5 %) ² **+ PAM (8 mg/L)** ¹ (5 µL = 2.5 %) ² + **Disper** ® (90 µL = 45 %) ² | **GSE** (5 µL = 2.5 %) ² **+ PAM (13mg/L) ¹** (5 µL =2.5%) ² = **+ Disper** ® (90 µL = 45 %) ² |
| *Staphylococcus aureus* ATCC 29213 | - | + | 0.78 | 0.15 | 0.08 | 0.3 | 0.3 |

| | | | | | | | |
|---|---|---|---|---|---|---|---|
| ¹ Final concentrations of PAM in the medium, i.e. 0.8 µg of PAM for C₃, 1.2 µg for C₄, 1.6 µg for C₅ and 2.6 µg for C₆, in the 200 µL of each appropriate pit. ² Percentage of each product in the 200 µL of each appropriate pit. ³ Positive control (*inoculum*): 100 % bacterial growth. ⁴ Negative control (sterile culture medium): 0 % bacterial growth. | | | | | | | |

The experimental results presented in table 3 show that the addition of the activating agent PAM according to the invention allows an increase in the inhibiting potency of grapefuit seed extract (GSE), even in the presence of an excipient of dispersant type (Disper®), on the microorganism *Staphylococcus aureus* ATCC 29213.

In composition C₃ according to the invention, the addition of the activating agent PAM, at a dose of 4 mg/L, allows an increase in the inhibiting potency of grapefruit seed extract (GSE), the MIC of which is divided by 5.2 (0.15 instead of 0.78).

In composition C₃ according to the invention, 0.8 µg of activating agent PAM are sufficient to increase the efficacy of 5 µL of GSE. Therefore, in this composition, an amount of PAM approximately 6 250 times lower than that of GSE is sufficient to increase the efficacy of this one.

In composition C₄ according to the invention, the addition of the activating agent PAM, at a dose of 6 mg/L, allows an increase in the inhibiting potency of grapefruit seed extract (GSE), the MIC of which is divided by 9.75 (0.08 instead of 0.78).

In composition C₄ according to the invention, 1.2 µg of activating agent PAM are sufficient to increase the efficacy of 5 µL of GSE. Therefore, in this composition, an amount of PAM approximately 4 166 times lower than that of GSE is sufficient to increase the efficacy of this one.

In compositions C₅ and C₆ according to the invention, the addition of the activating agent PAM, at doses of 8 mg/L and 13 mg/L, allows an increase in the inhibiting potency of grapefruit seed extract (GSE), the MIC of which is divided by 2.6 (0.3 instead of 0.78).

In composition C₅ according to the invention, 1.6 µg of activating agent PAM are sufficient to increase the efficacy of 5 µL of GSE. Therefore, in this composition, an amount of PAM approximately 3 125 times lower than that of GSE is sufficient to increase the efficacy of this one.

In composition C₆ according to the invention, 2.6 µg of activating agent PAM are sufficient to increase the efficacy of 5 µL of GSE. Therefore, in this composition, an amount of PAM approximately 1 923 times lower than that of GSE is sufficient to increase the efficacy of this one.

In composition C₅, the activating effect is observed with 8 mg/L of PAM. In composition C₆, the increase in the concentration of PAM from 8 mg/L to 13 mg/L (i.e. an incease by more than 50 %) does not yield an increase in the inhibiting potency which is 0.3 for both compositions C₅ and C₆: the MICs of C₅ and C₆ are 2.6 times lower than that of composition CC₂ (0.3 instead of 0.78).

In this example, although an amount of EPP lower than that in examples 1 and 2 (5 µL instead of 95 µL) and an excipient of dispersing type are used, the activating agent PAM according to the invention allows an increase in the efficacy of GSE.

These experimental results show that the inhibiting compositions according to the invention allow an increase in the inhibiting potency of the active ingredient used.

### EXAMPLE 4: activation of the inhibiting activity of grapefruit seed extract (Citrus paradisi) (GSE) on two nosocomial fungal strains

In addition to the compositions of control described above (F positive control and M negative control), the following compositions were prepared in order to show the action of the activating agent:
- a first comparative inhibiting composition CC₂, containing 100 µL of GSE,
- a second comparative inhibiting composition C₁, according to the invention, containing 95 µL of GSE and 5 µL of PAM, i.e. a total volume of 100 µL.

The final concentration of PAM in the medium, i.e. in the 200 µL of each appropriate pit, is 60 mg/L. Therefore there are 12 µg of PAM in these 200 µL.

100 µL of *inoculum* are added to each of the compositions described above.

For each of these compositions, the MIC of the active ingredient (GSE) was determined on two nosocomial fungal strains. The experimental results of these trials are presented in table 4 below.

**Table 4**

| **Nosocomial fungal strains** (100 µL of *inoculum*) | **Negative control** | **Positive control** | **MIC (% v/v) of the fungal strains by GSE alone or combined with PAM (60 mg/L) ¹** | |
|---|---|---|---|---|
| | | | **CC₂** | **C₁** |
| | **M ⁴** | **F ³** | **GSE** (100 µL = 50 %) ² | **GSE** (95 µL = 47.5 %) ² **+ PAM (60 mg/L) ¹** (5 µL = 2.5 %) ² |
| *Candida albicans* | - | + | 0.25 | 0.06 |
| *Malassezia furfur* | - | + | 0.50 | 0.05 |

| | | | | |
|---|---|---|---|---|
| ¹ Final concentration of PAM in the medium, i.e. 12 µg of PAM in the 200 µL of each appropriate pit. ² Percentage of each product in the 200 µL of each appropriate pit. ³ Positive control *(inoculum) :* 100 % fungal growth. ⁴ Negative control (sterile culture medium) : 0 % fungal growth. | | | | |

The experimental results presented in table 4 show that the addition of the activating agent PAM according to the invention allows an increase in the inhibiting potency of grapefuit seed extract (GSE), on two nosocomial fungal strains of *Candida albicans* and *Malassezia furfur.*

For the fungi *Candida albicans*, in composition C₁ according to the invention, the addition of the activating agent PAM, at a dose of 60 mg/L, allows an increase in the inhibiting potency of grapefruit seed extract (GSE), the MIC of which is divided by 4.16 (0.06 instead of 0.25).

For the fungi *Malassezia furfur*, in composition C₁ according to the invention, the addition of the activating agent PAM, at a dose of 60 mg/L, allows an increase in the inhibiting potency of grapefruit seed extract (GSE), the MIC of which is divided by 10 (0.05 instead of 0.50).

These experimental results show that the inhibiting composition according to the invention allows an increase in the inhibiting potency of the active ingredient used not only on bacteria, as demonstrated in the examples above, but also on fungi.

## Claims

1. Composition comprising at least one anti-infectious agent and one activating agent, said anti-infectious agent being an essential oil of oregano, thyme, savory or a grapefruit seed extract, and said activating agent being an extract made from dehydrated and partially sodium-free seawater.

2. Composition according to claim 1 for use in treating infectious diseases of viral, or bacterial or fungal origin, concerning the whole human or animal body.

## Patentansprüche

1. Zusammensetzung, wenigstens einen infektionshemmenden Wirkstoff und einen aktivierenden Wirkstoff umfassend, wobei der infektionshemmende Wirkstoff ein ätherisches Öl aus Oregano, Thymian, Bohnenkraut oder ein Grapefruitkern-Extrakt ist, und der aktivierende Wirkstoff ein Extrakt ist, hergestellt aus dehydriertem und teilweise natriumfreien Meerwasser.

2. Zusammensetzung nach Anspruch 1 zur Verwendung beim Behandeln von infektiösen Krankheiten von viralem, oder bakteriellem oder pilzartigem Ursprung, den gesamten menschlichen oder tierischen Körper betreffend.

## Revendications

1. Composition comprenant au moins un agent anti-infectieux et un agent d'activation, ledit agent anti-infectieux étant une huile essentielle d'origan, de thym, de sarriette, ou un extrait de pépins de pamplemousse, et ledit agent d'activation étant un extrait créé à partir d'eau de mer déshydratée et partiellement dépourvue de sodium.

2. Composition selon la revendication 1 pour son utilisation dans le traitement de maladies infectieuses d'origine virale, ou bactérienne ou fongique, concernant l'ensemble du corps humain ou animal.
